# EUROPEAN PATENT APPLICATION

(11) **EP 4 467 126 A1**
(43) Date of publication of application: **27.11.2024**
(21) Application number: 23760344.4
(22) Date of filing: 20.02.2023
(51) Int. Cl.: A61K 9/00, A61K 47/02, A61K 47/40, A61K 31/4725, A61P 19/00, A61P 29/00

(54) **INJECTABLE FORMULATION COMPRISING ISOXAZOLINE DERIVATIVE AND METHOD FOR PREPARING SAME**

(30) Priority: 23.02.2022 KR 20220023373
(71) Applicant: LG Chem, Ltd., Yeongdeungpo-gu Seoul 07336 (KR)
(72) Inventor: BAEK, Jaeuk, Daejeon 34122 (KR); KIM, Sung Won, Daejeon 34122 (KR); YOON, Jung Woon, Daejeon 34122 (KR)
(74) Representative: Hoffmann Eitle
(86) International application number: PCT/KR2023/002402
(87) International publication number: WO 2023/163470

(57) **Abstract**

The present invention relates to a preparation for injection comprising an isoxazoline derivative useful as a caspase inhibitor or a pharmaceutically acceptable salt thereof as an active pharmaceutical ingredient (API).

The preparation for injection according to the present invention includes the first formulation composed of a high-dose API and the second formulation including a reconstitution solution, so that it can be prepared by mixing the first formulation and the second formulation immediately before administration to a patient, and contains the Active-API stably. Therefore, there is an advantage that effective drug efficacy can be expected when administered to a patient.

## Description

### [Technical Field]

This application claims the benefit of priority based on Korean Patent Application No. 10-2022-0023373 filed on February 23, 2022, all the contents of which are incorporated herein as a part of this specification.

The present invention relates to a preparation for injection comprising an isoxazoline derivative useful as a caspase inhibitor or a pharmaceutically acceptable salt thereof as an active pharmaceutical ingredient (API) and a method for manufacturing thereof.

### [Background Art]

Caspase is a cysteine protease, and a caspase inhibitor is a compound that can control inflammation or apoptosis caused by the action of caspase by inhibiting the action of this caspase. Diseases for which this compound can be administered to eliminate or alleviate symptoms include osteoarthritis, rheumatoid arthritis, and degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes, gastric ulcer and the like.

As caspase inhibitors, various types of isoxazoline derivatives and prodrugs of the isoxazoline derivatives are known. Among the isoxazoline derivatives, (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide(Hereinafter, referred to as "Compound of Formula 1") has already proven its efficacy in liver disease caused by hepatitis virus, liver fibrosis, and nonalcoholic steatohepatitis (NASH).

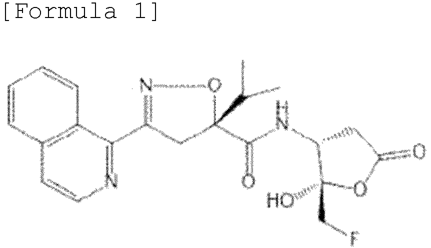

Recently, attempts have been made to apply the compound of Formula 1 to diseases related to bone and joint, such as osteoarthritis (OA), rheumatoid arthritis, degenerative arthritis, and destructive bone disorders.

In the treatment of the diseases related to bone and joint, a common drug administration method is to inject directly with a syringe into the joint cavity filled with synovial fluid. In the case of oral administration, the drug has a systemic effect, but systemic side effects are unavoidable. On the other hand, in the case of direct administration into the joint cavity, there are advantages in that a high concentration of the drug can be administered and systemic side effects can be minimized as well as a local therapeutic effect in which the drug is directly delivered due to local administration of the drug.

The compound of Formula 1 has been confirmed to have a therapeutic effect as the existing oral preparation for liver disease, but since the compound of Formula 1 is poorly soluble in water, it is difficult to formulate it in a preparation for injection.

Therefore, it is necessary to develop a high-concentration preparation for injection that can use the compound of Formula 1 as an injection.

### [Prior Art Documents]

### [Patent Document]

(Patent Document 1) Korean Patent No. 0774999(2007.11.02.) Isoxazoline derivatives and novel process for its preparation
(Patent Document 2) Korean Patent Application No. 2021-0102476(2021.08.04.) Use of caspase inhibitor for alleviating or treating osteoarthritis

### [Non-Patent Document]

(Non-Patent Document 1) Ratziu et al., 'A Phase 2, Randomized, Double-Blind, Placebo-Controlled Study of GS-9450 in Subjects with Nonalcoholic Steatohepatitis' HEPATOLOGY, Vol. 55, No. 2, 2012.

### [Disclosure]

### [Technical Problem]

Accordingly, the inventors of the present invention have conducted various studies to solve the above problems, and as a result, the problem to be solved by the present invention is to derive a preparation for injection comprising a high content of the compound of Formula 1 as an active pharmaceutical ingredient (API) .

In addition, in a preparation for injection containing a high content of the compound of Formula 1 as an active pharmaceutical ingredient (API), the problem to be solved by the present invention is to derive a freeze-dried preparation for injection capable of increasing the stability of the active pharmaceutical ingredient and allowing long-term storage.

### [Technical Solution]

The present invention provides a preparation for injection comprising a first formulation in the powder form including the following compound of Formula 1, a phosphate buffer, a solubilizing agent and a pH adjusting agent; and a second formulation including a reconstitution solution having a pH of 5 to 12:

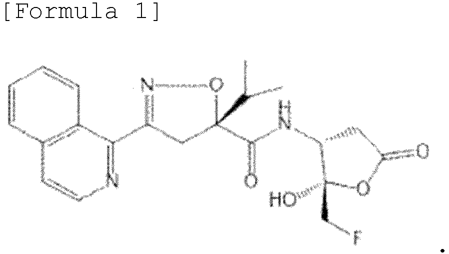

Further, the present invention provides a preparation for injection wherein the phosphate buffer is a mixture of Sodium phosphate dibasic heptahydrate and Sodium phosphate monobasic monohydrate.

Further, the present invention provides a preparation for injection wherein the content of the phosphate buffer is 1 to 15 mM based on the first formulation.

Further, the present invention provides a preparation for injection wherein the solubilizing agent is beta-cyclodextrin.

Further, the present invention provides a preparation for injection wherein the beta-cyclodextrin is any one selected from the group consisting of hydroxymethyl-beta-cyclodextrin, hydroxyethyl-beta-cyclodextrin and hydroxypropyl-beta-cyclodextrin.

Further, the present invention provides a preparation for injection wherein the content of the beta-cyclodextrin is 10 to 30 wt% based on the first formulation.

Further, the present invention provides a preparation for injection wherein the pH of the solution after reconstitution by mixing the first formulation and the second formulation is 5.0 to 7.0.

Further, the present invention provides a preparation for injection wherein the osmotic pressure of the solution after reconstitution by mixing the first formulation and the second formulation is 300 mOsm/kg to 400 mOsm/kg.

Further, the present invention provides a preparation for injection wherein the content of the compound of Formula 1 is 1 to 20 mg/ml based on the solution after reconstitution by mixing the first formulation and the second formulation.

Further, the present invention provides a method for manufacturing a preparation for injection comprising the following steps of: (S1) preparing a solvent having a pH of 10 to 12 including a phosphate buffer, a solubilizing agent and a basic pH adjusting agent; (S2) preparing a first solution having a pH of 6 to 8 by dissolving the following compound of Formula 1 in the prepared solvent; 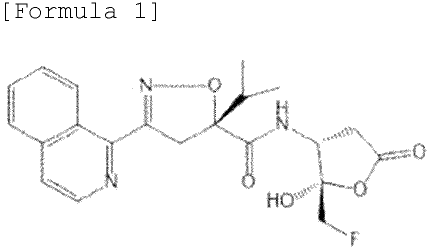 (S3) preparing a second solution having a pH of 1.0 to 3.0 by adding an acidic pH adjusting agent to the first solution; (S4) preparing a first formulation in the powder form by freeze-drying the second solution; and (S5) preparing a second formulation including a reconstitution solution having a pH of 5 to 12.

Further, the present invention provides method for manufacturing a preparation for injection wherein the content of the compound of Formula 1 is 1 to 20 mg/ml based on the solution after reconstitution.

Further, the present invention provides method for manufacturing a preparation for injection wherein the phosphate buffer is a mixture of Sodium phosphate dibasic heptahydrate and Sodium phosphate monobasic monohydrate.

Further, the present invention provides method for manufacturing a preparation for injection wherein the content of the phosphate buffer is 1 to 15 mM based on the solvent.

Further, the present invention provides method for manufacturing a preparation for injection wherein the solubilizing agent is beta-cyclodextrin.

Further, the present invention provides method for manufacturing a preparation for injection wherein the betacyclodextrin is any one selected from the group consisting of hydroxymethyl-beta-cyclodextrin, hydroxyethyl-beta-cyclodextrin and hydroxypropyl-betacyclodextrin.

Further, the present invention provides method for manufacturing a preparation for injection wherein the content of the beta-cyclodextrin is 10 to 30 wt% based on the solvent.

Further, the present invention provides a preparation for injection wherein the preparation for injection is for treating or preventing a disease selected from the group consisting of osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer.

Further, the present invention provides a preparation for injection wherein the disease is selected from the group consisting of osteoarthritis, rheumatoid arthritis, degenerative arthritis and destructive bone disorder.

Further, the present invention provides a preparation for injection which is used as a single-dose in a solution state in which the first formulation and the second formulation are mixed.

Further, the present invention provides a preparation for injection which is administered into the joint cavity after mixing the first formulation and the second formulation.

Further, the present invention provides a preparation for injection which is administered into the joint cavity within 30 minutes after mixing the first formulation and the second formulation.

Further, the present invention provides a preparation for injection which is administered once after mixing the first formulation and the second formulation.

Further, the present invention provides a preparation for injection comprising: a first formulation in the powder form including the following compound of Formula 1, a phosphate buffer, a solubilizing agent and a pH adjusting agent; and a second formulation including a reconstitution solution having a pH of 5 to 12: 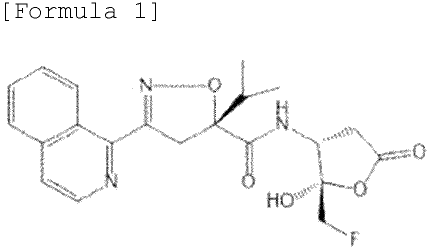 wherein, when the contents of the following compounds of Formula 2 and Formula 3 were measured within 30 minutes after mixing the first formulation and the second formulation, the content of the compound Formula 2 of the total contents of the compounds of Formula 2 and Formula 3 is 94% or more:

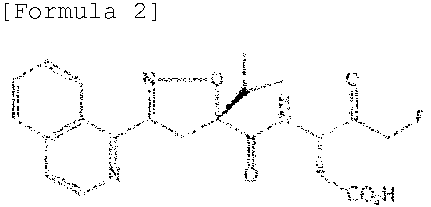

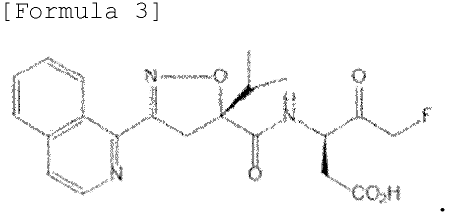

### [Advantageous Effects]

The preparation for injection according to the present invention includes a first formulation, which is a freeze-drying formulation, and a second formulation including a reconstitution solution, and the drug is prepared by mixing the first formulation and the second formulation immediately before administration to a patient, and then the drug is administered to the patient within 30 minutes. Therefore, a preparation for injection with a high content of Active-API can be provided.

In addition, it is possible to provide a freeze-drying preparation for injection capable of increasing the stability of active pharmaceutical ingredients (API) and allowing long-term storage.

### [Description of Drawings]

Fig. 1 is a diagram showing the results of measuring the API purity in the API mixed solution according to the stirring time.
Fig. 2 is (a) a diagram showing the TGA measurement result of the final API freeze-drying formulation prepared in Example 52, and (b) a diagram showing the DSC measurement result of the final API freeze-drying formulation prepared in Example 52.
Fig. 3 is a diagram showing the XRD pattern measurement results of the final API freeze-drying formulation prepared in Example 52.

### [Best Mode]

Hereinafter, the present invention will be described in detail.

All technical terms used in the present invention, unless defined otherwise, are used in the same meaning as commonly understood by those of ordinary skill in the art related to the present invention. In addition, although preferred methods or samples are described in this specification, those similar or equivalent thereto are also included in the scope of the present invention. The contents of all publications incorporated by reference herein are hereby incorporated by reference in their entirety.

Since the compound of Formula 1 of the present invention is hardly soluble in water, the solubility in solvents of various compositions was analyzed to find a solvent capable of dissolving the compound of Formula 1 in a high content.

As a result, it was confirmed that compound of Formula 1 was hardly soluble in acidic solvents of pH 4 or less among solvents containing a phosphate buffer, a solubilizing agent and a pH adjusting agent, but was dissolved well in weak acidic solvents of pH 6 or more and basic solvents of pH 7.5 or more. Further, it was confirmed that the compound of Formula 1 has the property of reducing the pH of a solution while being dissolved in a solvent.

In addition, it was found that when the compound of Formula 1 is dissolved in an aqueous solvent, the lactone ring is broken and converted into the compound of Formula 2 below. At this time, it was found that the compound of Formula 2 is in an active form, but the compound of Formula 2 is rapidly irreversibly converted into the compound of Formula 3, which is a structural isomer, in an aqueous solution, and unlike the compound of Formula 2, the compound of Formula 3 is in an inactive form that does not show the caspase inhibitory activity.

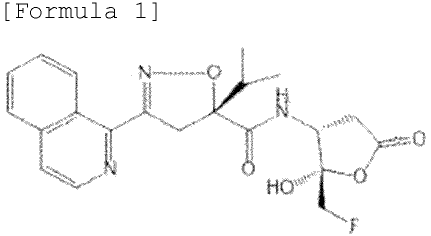

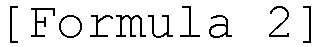

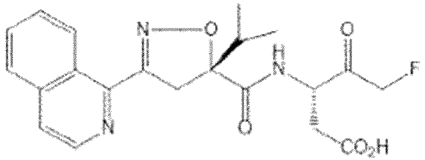

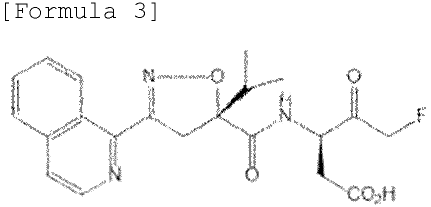

In addition, as a result of confirming the conversion rate from the compound of Formula 2 to the compound of Formula 3 according to the pH of the solvent, it was confirmed that the compound of Formula 3 was hardly produced in an acidic solvent, but the compound of Formula 3 was rapidly produced in a neutral or more basic solvent.

Therefore, in order to dissolve the compound of Formula 1, a basic solvent with a high pH should be used. However, it was found that as the compound of Formula 1 was dissolved, the solubility of the undissolved compound was lowered by lowering the pH of the solution, and the active form of the dissolved compound was rapidly changed to the inactive form in a basic solvent. This mechanism lowers the purity of the active ingredient in a preparation for injection using the compound of Formula 1 as an API, so there is a problem of affecting drug efficacy, and since the compound of Formula 1 lowers the pH of the solution as it dissolves, it is difficult to adjust the pH when preparing a final drug for subject administration.

Therefore, as a result of studying various preparation for injection, the present inventors completed a freeze-drying preparation for injection with improved long-term storage stability by preparing a solution containing the compound of Formula 1 as an acidified solution and then freeze-drying it.

### Method for manufacturing preparation for injection

According to one embodiment of the present invention, the method for manufacturing a preparation for injection comprises the following steps of:
(S1) preparing a solvent having a pH of 10 to 12 including a phosphate buffer, a solubilizing agent and a basic pH adjusting agent;
(S2) preparing a first solution having a pH of 6 to 8 by dissolving the following compound of Formula 1 in the prepared solvent; 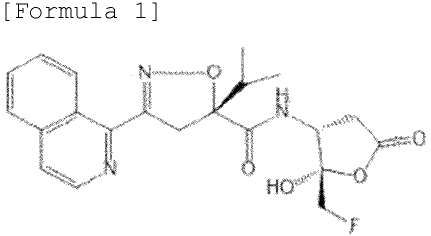
(S3) preparing a second solution having a pH of 1.0 to 3.0 by adding an acidic pH adjusting agent to the first solution;
(S4) preparing a first formulation in the powder form by freeze-drying the second solution; and
(S5) preparing a second formulation including a reconstitution solution having a pH of 5 to 12.

In one embodiment of the present invention, the method for manufacturing a preparation for injection may comprise a step of (S1) preparing a solvent having a pH of 10 to 12 including a phosphate buffer, a solubilizing agent and a basic pH adjusting agent.

The solvent containing the buffer, solubilizing agent and basic pH adjusting agent may be a composition of a solvent capable of dissolving the compound of Formula 1.

The solvent may include a buffer. The buffer may be a conventional buffer that can be used for a preparation for injection, and specific examples include, but are not limited to, phosphate buffer, Tris buffer and MES buffer. When using the phosphate buffer, it may be selected from the group consisting of sodium phosphate dibasic acid, sodium phosphate monobasic acid, potassium phosphate dibasic acid, potassium phosphate monobasic acid, and mixtures thereof. When using the Tris buffer, TRIZMA^{®} buffer can be used.

The buffer may have a concentration of 1 to 15 mM based on the solvent, specifically, 1 to 15 mM, 2 to 15 mM, 3 to 15 mM, 4 to 15 mM, 5 to 15 mM, 1 to 14 mM, 2 to 14 mM, 3 to 14 mM, 4 to 14 mM, 5 to 14 mM, 1 to 13 mM, 2 to 13 mM, 3 to 13 mM, 4 to 13 mM, 5 to 13 mM, 1 to 12 mM, 2 to 12 mM, 3 to 12 mM, 4 to 12 mM, 5 to 12 mM, 1 to 11 mM, 2 to 11 mM, 3 to 11 mM, 4 to 11 mM, 5 to 11 mM, 1 to 10 mM, 2 to 10 mM, 3 to 10 mM, 4 to 10 mM or 5 to 10 mM.

The solvent may include a solubilizing agent. The solubilizing agent may include cyclodextrin or a derivative thereof. The cyclodextrin can improve the dissolution stability of the compound of Formula 1 and affect the formation of a stable and uniform cake during freeze-drying.

The cyclodextrin is a cyclic oligosaccharide in which 6 to 12 glucose molecules are linked by alpha-1,4-glycosidic bonds, and includes alpha-cyclodextrin with 6 glucose molecules linked, betacyclodextrin with 7 glucose molecules linked, and gammacyclodextrin with 8 glucose molecules linked. Cyclodextrin that can be used in the present invention may include all cyclodextrin derivatives in addition to the above three types.

The cyclodextrin derivatives may include alpha-cyclodextrin, 2-hydroxypropyl-beta-cyclodextrin, sulfobutyl ether-beta-cyclodextrin and the like, and may specifically be 2-hydroxypropyl-beta-cyclodextrin.

The solubilizing agent may be contained 10 to 30 wt% based on the solvent. Specifically, it may be contained 10 to 30 wt%, 10.5 to 30 wt%, 11 to 30 wt%, 11.5 to 30 wt%, 12 to 30 wt%, 10 to 29.5 wt%, 10.5 to 29.5 wt%, 11 to 29.5 wt%, 11.5 to 29.5 wt%, 12 to 29.5 wt%, 10 to 29 wt%, 10.5 to 29 wt%, 11 to 29 wt%, 11.5 to 29 wt%, 12 to 29 wt%, 10 to 28.5 wt%, 10.5 to 28.5 wt%, 11 to 28.5 wt%, 11.5 to 28.5 wt%, 12 to 28.5 wt%, 10 to 28 wt%, 10.5 to 28 wt%, 11 to 28 wt%, 11.5 to 28 wt% or 12 to 28 wt%.

The solvent may include a basic pH adjusting agent. The basic pH adjusting agent can be used when preparing a solvent with a pH of 10 to 12, and for example, sodium hydroxide, potassium hydroxide, calcium hydroxide, magnesium hydroxide, ammonia, sodium bicarbonate, etc. can be used, but it is not limited to only these examples.

The basic pH adjusting agent has a concentration of 20 to 40 mM based on the solvent.

The pH of the solvent can be adjusted to 10 to 12 using the buffer and basic pH adjusting agent.

In one embodiment of the present invention, the method for manufacturing a preparation for injection may comprise a step of (S2) preparing a first solution having a pH of 6 to 8 by dissolving the following compound of Formula 1 in the prepared solvent:

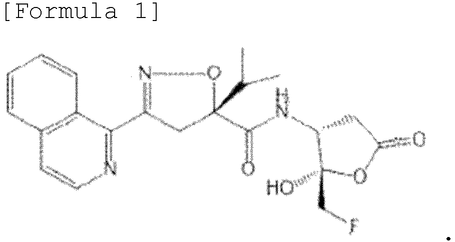

The compound of Formula 1 can be dissolved in a solvent with a pH of 10 to 12. As the compound of Formula 1 is dissolved in the solvent, the pH of the first solution is lowered, so the pH of the first solution in which the compound of Formula 1 and the solvent are mixed may be in the range of 6 to 8. If the pH of the mixed solution of the compound of Formula 1 is out of the range of 6 to 8, it may not be suitable for dissolving the API.

In one embodiment of the present invention, the method for manufacturing a preparation for injection may comprise a step of (S3) preparing a second solution having a pH of 1.0 to 3.0 by adding an acidic pH adjusting agent to the first solution.

The compound of Formula 1, an API, is dissolved in a solvent with a pH of 10 to 12, but the active form of the dissolved compound of Formula 1 rapidly changes to an inactive form in a basic solution. Therefore, the preparation of the second solution is to prevent the Active Form of the API from rapidly hydrolyzing in an aqueous solution and changing into the Inactive Form as the pH of the first solution containing the API increases.

In the preparation of the second solution, the API should not be precipitated, and the stability (purity) of the API should be maintained after preparation of the second solution. To satisfy these conditions, the pH of the second solution may be in the range of 1.0 to 3.0. Specifically, the pH of the second solution may be 1.0 to 3.0, 1.0 to 2.9, 1.0 to 2.8, 1.0 to 2.7, 1.0 to 2.6 or 1.0 to 2.5.

The acidic pH adjusting agent added to prepare the second solution may be hydrochloric acid, but is not limited thereto, and perchloric acid, nitric acid, sulfuric acid and the like that can satisfy the pH of the second solution of 1.0 to 3.0 may be used.

In one embodiment of the present invention, the method for manufacturing a preparation for injection may comprise a step of (S4) preparing a first formulation in the powder form by freeze-drying the second solution.

As used herein, the term "freeze-drying" refers to a process in which a material to be dried is first frozen, and then ice or a frozen solvent is removed by sublimation in a vacuum environment.

The freeze-drying may be performed by freezing at -80°C for 3 to 5 hours and then drying at 10 to 20 Pa for 10 to 50 hours.

The first formulation may be in the form of powder, dry powder or lyophilisate. The first formulation can be stored for a long period of time and is easy to formulate as an injection. Further, it does not harm the human body at all because there are no additives that may cause side effects.

In one embodiment of the present invention, the method for manufacturing a preparation for injection may comprise a step of (S5) preparing a second formulation including a reconstitution solution having a pH of 5 to 12.

As used herein, the term "reconstitution" means that the solid state is returned to the liquid state by adding water or a solution to dry powder such as freeze-dried powder, and the reconstitution solution according to the present invention refers to a solution for making the liquid state by adding to the first formulation.

The second formulation may include a reconstitution medium including sterile purified water, water for injection, physiological saline, or a combination thereof, and may include a reconstitution solution further comprising sodium hydroxide, potassium hydroxide, calcium hydroxide, magnesium hydroxide, or a combination thereof in the reconstitution medium.

The reconstitution solution may be a solution having a pH of 5 to 12. The preparation of the second formulation including the reconstitution solution having a pH of 5 to 12 is to reconstitute the first formulation prepared in powder form by freeze-drying the second solution having a pH of 1.0 to 3.0. This is to adjust the pH of the preparation for injection suitable for the body in order to prevent pain in the patient before administering the preparation for injection into the body.

The second formulation may further include other additives that do not affect the pH in addition to the reconstitution solution. The additives may further include preservatives, surfactants, and the like, but are not limited to, and may optionally include additives commonly used in the art. The preservative is to prevent secondary contamination, and a pharmaceutically acceptable preservative may be used. The surfactants include, for example, nonionic surfactants containing a polyoxyethylene group, but are not limited thereto, and a pharmaceutically acceptable surfactant may be used.

### Preparation for injection

According to one embodiment of the present invention, a preparation for injection comprising: a first formulation in the powder form including the following compound of Formula 1, a phosphate buffer, a solubilizing agent and a pH adjusting agent; and a second formulation including a reconstitution solution having a pH of 5 to 12 can be provided:

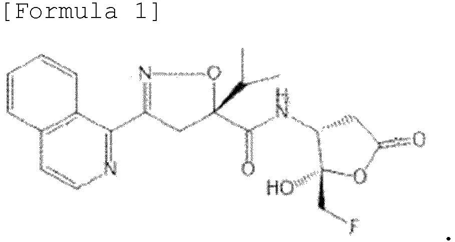

The first formulation may include a buffer, a solubilizing agent and a pH adjusting agent.

The buffer may be a conventional buffer that can be used for a preparation for injection, and specific examples include, but are not limited to, phosphate buffer, Tris buffer and MES buffer. When using the phosphate buffer, it may be selected from the group consisting of sodium phosphate dibasic acid, sodium phosphate monobasic acid, potassium phosphate dibasic acid, potassium phosphate monobasic acid, and mixtures thereof. When using the Tris buffer, TRIZMA^{®} buffer can be used.

The buffer may have a concentration of 1 to 15 mM based on the first formulation, specifically, 1 to 15 mM, 2 to 15 mM, 3 to 15 mM, 4 to 15 mM, 5 to 15 mM, 1 to 14 mM, 2 to 14 mM, 3 to 14 mM, 4 to 14 mM, 5 to 14 mM, 1 to 13 mM, 2 to 13 mM, 3 to 13 mM, 4 to 13 mM, 5 to 13 mM, 1 to 12 mM, 2 to 12 mM, 3 to 12 mM, 4 to 12 mM, 5 to 12 mM, 1 to 11 mM, 2 to 11 mM, 3 to 11 mM, 4 to 11 mM, 5 to 11 mM, 1 to 10 mM, 2 to 10 mM, 3 to 10 mM, 4 to 10 mM or 5 to 10 mM.

The solubilizing agent may include cyclodextrin or a derivative thereof. The cyclodextrin can improve the dissolution stability of the compound of Formula 1 and affect the formation of a stable and uniform cake during freeze-drying.

The cyclodextrin is a cyclic oligosaccharide in which 6 to 12 glucose molecules are linked by alpha-1,4-glycosidic bonds, and includes alpha-cyclodextrin with 6 glucose molecules linked, betacyclodextrin with 7 glucose molecules linked, and gammacyclodextrin with 8 glucose molecules linked. Cyclodextrin that can be used in the present invention may include all cyclodextrin derivatives in addition to the above three types.

The cyclodextrin derivatives may include alpha-cyclodextrin, 2-hydroxypropyl-beta-cyclodextrin, sulfobutyl ether-betacyclodextrin and the like, and may specifically be 2-hydroxypropyl-beta-cyclodextrin.

The solubilizing agent may be contained in an amount of 10 to 30 wt% based on the first formulation. Specifically, it may be contained in an amount of 10 to 30 wt%, 10.5 to 30 wt%, 11 to 30 wt%, 11.5 to 30 wt%, 12 to 30 wt%, 10 to 29.5 wt%, 10.5 to 29.5 wt%, 11 to 29.5 wt%, 11.5 to 29.5 wt%, 12 to 29.5 wt%, 10 to 29 wt%, 10.5 to 29 wt%, 11 to 29 wt%, 11.5 to 29 wt%, 12 to 29 wt%, 10 to 28.5 wt%, 10.5 to 28.5 wt%, 11 to 28.5 wt%, 11.5 to 28.5 wt%, 12 to 28.5 wt%, 10 to 28 wt%, 10.5 to 28 wt%, 11 to 28 wt%, 11.5 to 28 wt% or 12 to 28 wt%.

The pH adjusting agent may include a basic pH adjusting agent and an acidic pH adjusting agent. The pH adjusting agent is as described above.

The first formulation may be in the form of a powder, dry powder or lyophilisate. The first formulation can be stored for a long period of time and is easy to formulate as an injection. Further, it does not harm the human body at all because there are no additives that may cause side effects.

The second formulation may include a reconstitution solution having a pH of 5 to 12. The reconstitution solution is as described above.

In the preparation for injection according to one embodiment of the present invention, the pH of the solution after reconstitution by mixing the first formulation and the second formulation can be 5.0 to 7.0. If the pH of the solution after the reconstitution is out of the range of 5.0 to 7.0, the patient may feel pain when administering the preparation for injection to the patient, and thus may be unsuitable for use as a preparation for injection.

In the preparation for injection according to one embodiment of the present invention, the osmotic pressure of the solution after reconstitution by mixing the first formulation and the second formulation may be 300 mOsm/kg to 400 mOsm/kg. If the osmotic pressure of the solution after the reconstitution is out of the range of 300 mOsm/kg to 400 mOsm/kg, the patient may feel pain when administering the preparation for injection to the patient, and thus may be unsuitable for use as a preparation for injection.

In the preparation for injection according to one embodiment of the present invention, the content of the compound of Formula 1 may vary depending on the condition of the patient to be administered, the degree of treatment desired, and the like.

The preparation for injection may include the compound of Formula 1 in an amount of 1 to 20 mg/ml based on the solution after reconstitution by mixing the first formulation and the second formulation. Specifically, the content of the compound of Formula 1 may be 1 to 20 mg/ml, 5 to 20 mg/ml, 10 to 20 mg/ml, 1 to 15 mg/ml, 5 to 15 mg/ml, 10 to 15 mg/ml, 5 mg/ml, 10 mg/ml, 15 mg/ml or 20 mg/ml based on the solution after reconstitution, which may be preferred as a single dose.

When the content of the compound of Formula 1 is at a low concentration of less than 1 mg/ml, a large amount of the injection solution is injected to exhibit a sufficient therapeutic effect, which may cause difficulties in administering to the affected area of the patient, and when the content of the compound of Formula 1 is a high concentration of exceeding 20 mg/ml, the API may precipitate when the solvent is dissolved or cause pain when administered to the patient's affected area.

The preparation for injection according to one embodiment of the present invention may be in a transparent state in which the compound of Formula 1 is completely dissolved by mixing the first formulation including the compound of Formula 1 with the second formulation.

The preparation for injection according to one embodiment of the present invention can be used by reconstituting the first formulation containing the compound of Formula 1 with the second formulation containing the reconstitution solution immediately before administration to the patient and administering the solution to the affected area of the patient after the reconstitution.

The preparation for injection according to one embodiment of the present invention is used by mixing the first formulation and the second formulation immediately before administration to the patient, so it may not additionally contain excipients for preservation such as antioxidants and preservatives, but in the case of the second formulation including the reconstitution solution, a preservative to prevent secondary contamination may be included.

The present invention provides a method for treating or preventing caspase-related diseases using the preparation for injection of the present invention.

The term "treating" means stopping or delaying the progression of the disease when used in a subject showing symptoms of disease, and the "preventing" means stopping or delaying the signs of a disease when used in a high-risk subject who does not show symptoms of disease.

The "caspase-related disease" of the present invention refers to a disease that can be treated or prevented by inhibiting caspase, for example, osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer, but is not limited thereto the above disease.

The preparation for injection of the present invention can be administered to a subject in need of treatment or prevention of a caspase-associated disease to treat or prevent the disease in the subject.

The term "administration" or "administering" as used herein refers to a method of administering by injection a dose of the preparation for injection of the present invention to a vertebrate including mammals, birds, fish or amphibians or non-vertebrate animal. Preferred administration methods may be selected from conventional injection methods such as intravascular injection, subcutaneous injection, transdermal injection, intramuscular injection, spinal injection, and intradermal injection, depending on the type of disease to be treated, the site of the disease, and the severity of the disease. In particular, injection into the joint cavity can be selected for diseases related to bone joints such as osteoarthritis, rheumatoid arthritis, degenerative arthritis, or destructive bone disorders.

In the case of injection into the joint cavity using the preparation for injection of the present invention, administration may be guided using an imaging method using ultrasound or the like, or a local anesthetic may be administered in advance or in combination.

The term "subject" as used herein refers to a human or non-human mammal, such as a dog, cat, mouse, rat, cow, sheep, pig, goat, non-human ape, or bird. In some embodiments, the subject is a human.

The preparation for injection of the present invention is administered to the subject within 30 minutes after making a mixed solution by mixing the first formulation and the second formulation. Therefore, the preparation for injection of the present invention is intended for single-dose for singe patient infusion or administration.

In addition, the preparation for injection of the present invention may be additionally administered in combination with drugs such as analgesics, anti-inflammatory drugs, and steroids. Specifically, it can be administered in combination with drugs such as non-steroidal anti-inflammatory drugs (NSAIDs) selected from ibuprofen, naproxen, aspirin, acetaminophen, indomethacin, diclofenac taken orally or at the site of the lesion, meloxicam, celecoxib, piroxicam, etodolac, nabumetone, lumiracoxib, valdecoxib, etoricoxib, parecoxib, fenoprofen, oxaprozin, mefenamic acid, diflunisal, fluvirofen and ketoprofen; corticoid drugs such as prednisone, methylprednisolone; narcotic pain relievers such as codeine, fentanyl, morphine and meperidine; or injection of hyaluronic acid derivative.

The present invention provides a kit comprising the preparation for injection of the present invention. In one embodiment of the present invention, the kit may include a preparation for injection comprising the first formulation in powder form containing the compound of Formula 1 of the present invention, and the second formulation containing a reconstitution solution; an administration system such as one or more syringes for administering the agent; and instructions for using the kit, including how to mix the first formulation and the second formulation, and directions for treating patients.

In one embodiment of the present invention, the kit may include a label stating that the formulation and contents as described herein are administered to a patient suffering from a disease associated with bone joints such as osteoarthritis, rheumatoid arthritis, degenerative arthritis and destructive bone disorder. Herein, selecting a dosage suitable for the patient is within the knowledge of a person skilled in the art in consideration of the patient's symptoms, age, etc.

Unless indicated otherwise, all numbers used in the specification and claims are to be understood as being modified in all instances by the term "about," whether or not so stated. It should also be understood that the precise numerical values used in the specification and claims form additional embodiments of the disclosure. Efforts have been made to ensure the accuracy of the numerical values disclosed in the Examples. Any measured numerical value, however, can inherently contain certain errors resulting from the standard deviation found in its respective measuring technique.

Various evaluations in the Examples and the Comparative Examples were conducted as follow.

### Preparation Example

(R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide), a compound of Formula 1 used as API in the present invention (hereinafter referred to as "API") was prepared according to KR 10-0774999.

### Test Example 1: Confirmation of API dissolution according to solvent composition, pH of solvent, and stability of API in API mixed solution (Examples 1 to 16)

A solvent having the composition shown in Table 1 below for preparing a mixed solution of API having a concentration of 10 mg/ml by dissolving the API was prepared. As shown in Table 1 below, a solvent containing 0 to 100 mM sodium phosphate buffer (PB), 24 to 36 mM sodium hydroxide (NaOH) and 10 to 30% (w/v) hydroxypropyl beta cyclodextrin (HPβCD) was prepared.

Sodium phosphate buffer was prepared by mixing sodium phosphate dibasic heptahydrate and sodium phosphate monobasic monohydrate to have a pH of 7.4. For HPβCD, a parenteral grade reagent with a molar substitution range of 0.81 to 0.99 and a molecular weight of about 1500 g/mol was used.

As shown in Table 1 below, 10 mg of API was added to 1ml of the solvent prepared in Examples 1 to 16, and stirred at about 800 rpm for 30 minutes to prepare an API mixed solution.

**[Table 1]**

| **Example** | **Solvent Composition** | | | **Solvent pH** |
|---|---|---|---|---|
| | **PB (mM)** | **NaOH (mM)** | **HPβCD (wt%)** | |
| **Example 1** | 0 | 30 | 10 | 11.95 |
| **Example 2** | 100 | 25 | 10 | 7.95 |
| **Example 3** | 0 | 25 | 30 | 11.51 |
| **Example 4** | 100 | 30 | 30 | 9.57 |
| **Example 5** | 7.5 | 24 | 10 | 11.83 |
| **Example 6** | 7.5 | 30 | 10 | 11.95 |
| **Example 7** | 7.5 | 36 | 10 | 12.03 |
| **Example 8** | 10 | 24 | 15 | 11.49 |
| **Example 9** | 10 | 24 | 12.5 | 11.56 |
| **Example 10** | 10 | 24 | 10 | 11.71 |
| **Example 11** | 10 | 30 | 15 | 11.61 |
| **Example 12** | 10 | 30 | 12.5 | 11.65 |
| **Example 13** | 10 | 30 | 10 | 11.82 |
| **Example 14** | 10 | 36 | 15 | 11.72 |
| **Example 15** | 10 | 36 | 12.5 | 11.78 |
| **Example 16** | 10 | 36 | 10 | 11.94 |

Table 2 below is the result of confirming the pH of the API mixture solution obtained by adding and mixing the API in the solvent of the composition described in Table 1 above and the API stability (purity) in the API mixture solution after 30 minutes of mixing.

Since the active form of the API of Formula 1 is rapidly hydrolyzed in an aqueous solution and changed to the inactive form that has no activity, in order to confirm the stability of the API (purity or active form content in the API mixture solution) after 30 minutes of preparation of the API mixture solution, the peak area (%) was measured using HPLC.

**[Table 2]**

| **Example** | **Dissolution** | **Solution pH after API mixing** | **Peak Area (%)** | |
|---|---|---|---|---|
| | | | **Active Form** | **Inactive Form** |
| **Example 1** | Dissolved | 8.15 | 90.7 | 7.19 |
| **Example 2** | Dissolved | 7.1 | 96.2 | 3.8 |
| **Example 3** | Not dissolved | - | - | - |
| **Example 4** | Dissolved | 7.35 | 96.2 | 3.8 |
| **Example 5** | Not dissolved | 7.06 | - | - |
| **Example 6** | Not dissolved | 7.73 | - | - |
| **Example 7** | Dissolved | 9 | - | - |
| **Example 8** | Dissolved | 6.88 | 94.5 | 5.5 |
| **Example 9** | Dissolved | 6.9 | 94.4 | 5.6 |
| **Example 10** | Dissolved | 6.95 | 93.56 | 6.23 |
| **Example 11** | Dissolved | 7.24 | 85.2 | 6.98 |
| **Example 12** | Dissolved | 8.59 | 89.0 | 8.6 |
| **Example 13** | Dissolved | 8.01 | 86.04 | 9.03 |
| **Example 14** | Dissolved | 9.04 | - | - |
| **Example 15** | Dissolved | 9.03 | - | - |
| **Example 16** | Dissolved | 9.39 | 79.26 | 8.82 |

It was confirmed that solvents in which the API is not dissolved (Examples 3, 5 and 6), solvent in which the pH of the API mixed solution is out of the range of 6 to 8 (Examples 1, 7 and 12 to 16), or solvents in which the API purity (peak area %) is less than 94% after 30 minutes of preparation of the API mixture solution (Examples 1 and 10 to 16) are not suitable for dissolving the API of the present invention. Therefore, it was confirmed that the solvents prepared in Examples 2, 4, 8 and 9 are suitable solvents for dissolving the API.

In Examples 1 to 4, it was confirmed that since the solvent containing the phosphate buffer (Examples 2 and 4) is easy to control the pH, the solvents are suitable for dissolving the API or maintaining the purity of the API at 94% or higher after 30 minutes of preparing the API mixed solution.

When the concentration of the phosphate buffer is 10 mM or less and the concentration of NaOH is 30 to 36 mM (Examples 6, 7 and 11 to 16), the pH of the API mixed solution is 7.2 or higher. Therefore, it is desirable to lower the NaOH concentration to an appropriate level. This has a problem in that the purity of the API is lowered because the active form of the API of Formula 1 is rapidly hydrolyzed in an aqueous solution and changed to the inactive form as the pH of the mixed solution of the API is higher.

On the other hand, when the concentration of the phosphate buffer is 10 mM or less, the NaOH concentration is 24 mM, and the content of HPβCD is 12.5 wt% to 15 wt% (Examples 8 and 9), the pH of the API mixed solution was in the range of 6 to 7, and the purity of the API was also maintained at 94% or higher. Therefore, it was confirmed that the solvents prepared in Examples 8 and 9 are suitable for preparing the API mixed solution.

### Test Example 2: Preparation of acidified mixed solution and Confirmation of API stability in acidified mixed solution (Examples 17 to 22)

An acidified mixed solution was prepared by adding an acid (6 N HCl) to the previously prepared API mixture solutions containing the solvents of Examples 2, 4, 8 and 9.

The preparation of the acidified mixed solution is to prevent the active form of the API of Formula 1 from rapidly hydrolyzing in an aqueous solution and changing into the inactive form as the pH of the mixed solution of API increases.

As shown in Table 3 below, after adding 60 to 150 mM HCl using 6 N HCl as an acidifying solution to the API mixed solution containing the solvents prepared in Examples 2, 4, 8 and 9 above, an acidified mixed solution was prepared.

**[Table 3]**

| **Example** | **API mixed solution** | | | **Acidifying solution** | | |
|---|---|---|---|---|---|---|
| | **Solvent composition** | **solvent volume (ml)** | **API content (mg)** | **Acidifying solution composition** | **Add volume (µl)** | **HCl concentration after addition (mM)** |
| **Example 17** | Example 2 | 1 | 10 | 6N HCl | 10 | 60 |
| **Example 18** | Example 4 | 1 | 10 | 6N HCl | 10 | 60 |
| **Example 19** | Example 4 | 1 | 10 | 6N HCl | 20 | 120 |
| **Example 20** | Example 4 | 1 | 10 | 6N HCl | 25 | 150 |
| **Example 21** | Example 8 | 1 | 10 | 6N HCl | 10 | 60 |
| **Example 22** | Example 9 | 1 | 10 | 6N HCl | 10 | 60 |

Table 4 below is the result of confirming the pH of the acidified mixed solution prepared by adding the acidifying solution to the API mixed solution described in Table 3, API precipitation in the acidified mixed solution, and API stability (purity).

**[Table 4]**

| **Example** | **API Precipitation after preparing acidified solution** | **pH of Acidified mixed solution** | **Peak area (%)** | | **Peak area (%)** | |
|---|---|---|---|---|---|---|
| | | | **Right after preparation of acidified mixed solution** | | **1 day after preparation of acidified mixed solution** | |
| | | | **Active Form** | **Inactive Form** | **Active Form** | **Inactive Form** |
| **Example 17** | Not precipitated | 5.99 | 96.2 | 3.8 | 59.7 | 26.7 |
| **Example 18** | Not precipitated | 6.07 | 95.9 | 4.1 | 60.1 | 26.1 |
| **Example 19** | Not precipitated | 2.63 | 96.5 | 3.5 | 95.3 | 4.7 |
| **Example 20** | Not precipitated | 2.03 | 96.1 | 3.9 | 95.9 | 4.1 |
| **Example 21** | Not precipitated | 1.75 | 94.5 | 5.5 | 94.4 | 5.6 |
| **Example 22** | Not precipitated | 1.74 | 94.4 | 5.6 | 94.1 | 5.9 |

From the results of Table 4, it was confirmed that the API was not precipitated in the acidified mixed solution containing the API prepared in Examples 17 to 22 and was dissolved in the solution.

In Examples 17 and 18, the pH of the acidified mixed solution containing the API was about 6.0, and the purity dropped rapidly after preparing the acidified mixed solution, and decreased to about 60% after 1 day of preparing the acidified mixed solution. Through this, it was confirmed that when the pH of the acidified mixed solution containing the API was about 6.0, it was difficult for the API to stably present.

In Examples 19 and 20, the pH of the acidified mixed solution containing the API was about 2.0 to 2.7, and it remained stable with little change in purity after 1 day of preparing the acidified solution. However, it was confirmed that the lower the pH of the acidified mixed solution, the less change in purity, and thus the API was more stable.

In Examples 21 and 22, the pH of the acidified mixed solution containing the API was about 1.7, and after 1 day of preparing the acidified mixed solution, the purity remained stable with little change.

Therefore, when the pH of the acidified solution containing the API was 1.0 to 2.0, it was confirmed that the API was stably present.

### Test Example 3: Preparation of freeze-drying formulation (Examples 23 to 25)

The previously prepared solutions of Examples 20 to 22 were freeze-dried. The freeze-drying was performed under the conditions described in Table 5 below. That is, the freeze-drying was performed by freezing at -80°C for 3 hours and then drying at 10 to 20 Pa for 16 hours or 40 hours.

**[Table 5]**

| **Example** | **Acidification** | **Freeze-drying** | |
|---|---|---|---|
| | **Acidified solution** | **Freezing condition** | **Drying condition** |
| **Example 23** | Example 20 | -80°C, 3 hr | 10-20 Pa, 16 hr |
| **Example 24** | Example 21 | | 10-20 Pa, 40 hr |
| **Example 25** | Example 22 | | |

In the properties of the API freeze-drying formulation prepared in Examples 23 to 25 above, a white cake was uniformly formed, confirming that freeze-drying was successful.

### Test Example 4: Preparation of reconstitution solution, and confirmation of reconstitution time, pH and osmotic pressure after reconstitution and stability after reconstitution (Examples 26 to 29)

In order to reconstitute the previously prepared freeze-drying formulations of Examples 23 to 25, a reconstitution solution was prepared as shown in Table 6 below. The add volume of the reconstitution solvent is the value obtained by calculating the volume of water drained after freeze-drying in Examples 23 to 25 above. After adding the reconstitution solvent to the freeze-drying formulation, a reconstitution solution was prepared by shaking it rapidly.

**[Table 6]**

| **Example** | **Preparation of reconstitution solution** | | |
|---|---|---|---|
| | **Subject of reconstitution** | **reconstitution solution composition** | **Add volume of reconstitution solution (ml)** |
| **Example 26** | Example 23 | 200 mM NaOH | 0.75 |
| **Example 27** | Example 23 | 240 mM NaOH | 0.75 |
| **Example 28** | Example 24 | 58.43 mM NaOH | 0.89 |
| **Example 29** | Example 25 | 59.34 mM NaOH | 0.91 |

Table 7 below shows the results of measuring the time required for the reconstitution solution prepared in Examples 26 to 29 to become a transparent solution upon reconstitution, the pH and osmotic pressure of the solution after reconstitution, and the purity of the solution after reconstitution. Here, the purity of the solution after reconstitution was confirmed through UPLC.

**[Table 7]**

| **Example** | **Reconstitution time** | **Solution after reconstitution** | | **Peak area (%)** | | | |
|---|---|---|---|---|---|---|---|
| | | | | **Right after reconstitution** | | **30 min after reconstitution** | |
| | | **pH** | **Osmotic pressure (mOsm/kg)** | **Active Form** | **Inactive Form** | **Active Form** | **Inactive Form** |
| **Example 26** | 1 min 30 sec | 6.51 | 1213 | 94.2 | 5.2 | 93.1 | 6.3 |
| **Example 27** | 1 min | 6.86 | 1249 | 93.6 | 5.3 | 94.0 | 5.8 |
| **Example 28** | Within 1 min | 6.49 | 365 | 95.2 | 4.1 | 94.1 | 5.2 |
| **Example 29** | Within 1 min | 6.74 | 308 | 94.4 | 4.5 | 93.3 | 5.6 |

From the results of Table 7, it was confirmed that the pH of the solution after reconstitution prepared by Examples 26 to 29 was 6.4 to 6.9, which was suitable for use as an injection.

However, the preferred osmotic pressure range of the formulation for use as an injection is 300 to 400 mOsm/kg, and patients may feel pain if it falls below or exceeds this range. Therefore, when these criteria were applied, it was confirmed that Examples 28 and 29, in which the osmotic pressure of the solution after reconstitution was 308 mOsm/kg and 365 mOsm/kg, respectively, were preferable as an injection formulation containing 10 mg/ml API.

### Test Example 5: Confirmation of API dissolution according to composition, pH of solvent and API stability in API mixed solution (Examples 30 to 34)

A solvent having the composition shown in Table 8 below for preparing a mixed solution of API having a concentration of 15 mg/ml by dissolving the API was prepared. As shown in Table 8 below, a solvent containing 5 to 10 mM sodium phosphate buffer (PB), 35 to 40 mM sodium hydroxide (NaOH) and 17.5 to 20.0 %(w/v) hydroxypropyl beta cyclodextrin (HPβCD) was prepared.

Sodium phosphate buffer was prepared by mixing sodium phosphate dibasic heptahydrate and sodium phosphate monobasic monohydrate to have a pH of 7.4. For HPβCD, a parenteral grade reagent with a molar substitution range of 0.81 to 0.99 and a molecular weight of about 1500 g/mol was used.

As shown in Table 1 below, 15 mg of API was added to 1 ml of the solvent prepared in Examples 30 to 34, and stirred at about 800 rpm for 30 minutes to prepare an API mixed solution.

**[Table 8]**

| **Example** | **Solvent Composition** | | | **solvent pH** |
|---|---|---|---|---|
| | **PB (mM)** | **NaOH (mM)** | **HPβCD (wt%)** | |
| **Example 30** | 10 | 37.5 | 20 | 11.82 |
| **Example 31** | 10 | 40 | 20 | 11.84 |
| **Example 32** | 5 | 35 | 17.5 | 11.85 |
| **Example 33** | 5 | 37.5 | 17.5 | 11.88 |
| **Example 34** | 5 | 40 | 17.5 | 11.94 |

Table 9 below is the result of confirming the pH of the API mixture solution obtained by adding and mixing the API in the solvent of the composition described in Table 8 above and the API stability (purity) in the API mixture solution after 30 minutes of mixing.

Since the active form of the API of Formula 1 is rapidly hydrolyzed in an aqueous solution and changed to the inactive form that has no activity, in order to confirm the stability of the API (purity or active form content in the API mixture solution) after 30 minutes of preparation of the API mixture solution, the peak area (%) was measured using HPLC.

**[Table 9]**

| **Example** | **Dissolution** | **Solution pH after API mixing** | **Peak Area (%)** | |
|---|---|---|---|---|
| | | | **Active Form** | **Inactive Form** |
| **Example 30** | Dissolved | 7.04 | 96.5 | 3.3 |
| **Example 31** | Dissolved | 7.13 | 96.2 | 3.6 |
| **Example 32** | Dissolved | 6.13 | 95.5 | 3.9 |
| **Example 33** | Dissolved | 6.49 | 96.2 | 3.5 |
| **Example 34** | Dissolved | 7.17 | 94.2 | 4.7 |

From the result of Table 9 above, it was confirmed that the solvents prepared by Examples 30 to 34 are suitable for dissolving the API.

It was confirmed that the pH of the API mixture solution increased as the concentration of the phosphate buffer was 10 mM or less and the NaOH concentration increased (Examples 31 and 34) . It is preferable to lower the NaOH concentration to an appropriate level. This has a problem in that the purity of the API is lowered because the active form of the API of Formula 1 is rapidly hydrolyzed in an aqueous solution and changed to the inactive form as the pH of the mixed solution of the API is higher.

### Test Example 6: Preparation of acidified mixed solution and Confirmation of API stability in acidified mixed solution (Examples 35 to 39)

An acidified mixed solution was prepared by adding an acid (6 N HCl) to the previously prepared API mixture solutions containing the solvents of Examples 30 to 34.

The preparation of the acidified mixed solution is to prevent the active form of the API of Formula 1 from rapidly hydrolyzing in an aqueous solution and changing into the inactive form as the pH of the mixed solution of API increases.

As shown in Table 10 below, after adding 50 to 60 mM HCl using 6 N HCl as an acidifying solution to the API mixed solution containing the solvents prepared in Examples 30 to 34 above, an acidified mixed solution was prepared.

**[Table 10]**

| **Example** | **API mixed solution** | | | **Acidifying solution** | | |
|---|---|---|---|---|---|---|
| | **Solvent** | **Solvent volume (ml)** | **API content (mg)** | **Acidifying solution composition** | **Add volume (µl)** | **HCl concentration after addition (mM)** |
| **Example 35** | Example 30 | 1 | 15 | 6N HCl | 10 | 60 |
| **Example 36** | Example 31 | 1 | 15 | 6N HCl | 10 | 60 |
| **Example 37** | Example 32 | 1 | 15 | 6N HCl | 8.3 | 50 |
| **Example 38** | Example 33 | 1 | 15 | 6N HCl | 8.3 | 50 |
| **Example 39** | Example 34 | 1 | 15 | 6N HCl | 8.3 | 50 |

Table 11 below is the result of confirming the pH of the acidified mixed solution prepared by adding the acidifying solution to the API mixed solution described in Table 10, API precipitation in the acidified mixed solution, and API stability (purity).

**[Table 11]**

| **Example** | **API Precipitation after preparing acidified solution** | **pH of Acidified mixed solution** | **Peak area (%)** | | **Peak area (%)** | |
|---|---|---|---|---|---|---|
| | | | **Right after preparation of acidified mixed solution** | | **1 day after preparation of acidified mixed solution** | |
| | | | **Active Form** | **Inactive Form** | **Active Form** | **Inactive Form** |
| **Example 35** | Not precipitated | 1.86 | 96.4 | 3.5 | 96.1 | 3.8 |
| **Example 36** | Not precipitated | 1.94 | 96.1 | 3.7 | 95.8 | 3.9 |
| **Example 37** | Not precipitated | 2.04 | 95.4 | 4.0 | - | - |
| **Example 38** | Not precipitated | 2.06 | 96.1 | 3.5 | - | - |
| **Example 39** | Not precipitated | 2.15 | 94.1 | 4.8 | - | - |

From the results of Table 11, it was confirmed that the API was not precipitated in the acidified mixed solution containing the API prepared in Examples 35 to 39 and was dissolved in the solution. In Examples 35 and 36, the pH of the acidified mixed solution containing the API was about 1.8 to 2.0, and remained stable with little change in purity after 1 day of preparation of the acidified mixed solution.

In Examples 37 to 39, the pH of the acidified solution containing the API was about 2.0 to 2.2, which was low, but a slight change in purity occurred after 1 day of preparation of the acidified solution.

Therefore, when the pH of the acidified solution containing the API was 2.0 or lower, it was confirmed that the API was stably present.

### Test Example 7: Preparation of freeze-drying formulation (Examples 40 to 44)

The previously prepared solutions of Examples 35 to 39 were freeze-dried. The freeze-drying was performed under the conditions described in Table 12 below. That is, the freeze-drying was performed by freezing at -80°C for 3 hours and then drying at 10 to 20 Pa for 40 hours.

**[Table 12]**

| **Example** | **Acidification** | **freeze-drying** | |
|---|---|---|---|
| | **Acidified Solution** | **Freezing condition** | **Drying condition** |
| **Example 40** | Example 35 | -80 °C, 3 hr | 10-20 Pa, 40 hr |
| **Example 41** | Example 36 | | |
| **Example 42** | Example 37 | | |
| **Example 43** | Example 38 | | |
| **Example 44** | Example 39 | | |

In the properties of the API freeze-drying formulation prepared in Examples 40 to 44, an opaque white cake was uniformly formed, confirming that freeze-drying was successful.

### Test Example 8: Preparation of reconstitution solution, and confirmation of reconstitution time, pH and osmotic pressure of solution after reconstitution and stability after reconstitution (Examples 45 to 49)

In order to reconstitute the previously prepared freeze-drying formulations of Examples 23 to 25, a reconstitution solution was prepared as shown in Table 13 below. The add volume of the reconstitution solvent is the value obtained by calculating the volume of water drained after freeze-drying in Examples 23 to 25 above. After adding the reconstitution solvent to the freeze-drying formulation, a reconstitution solution was prepared by shaking it rapidly.

**[Table 13]**

| **Example** | **Preparation of reconstitution solution** | | |
|---|---|---|---|
| | **Subject of reconstitution** | **Reconstitution solution composition** | **Add volume of reconstitution solution (ml)** |
| **Example 45** | Example 40 | 70.59 mM NaOH | 0.85 |
| **Example 46** | Example 41 | 70.59 mM NaOH | 0.85 |
| **Example 47** | Example 42 | 51.72 mM NaOH | 0.87 |
| **Example 48** | Example 43 | 51.72 mM NaOH | 0.87 |
| **Example 49** | Example 44 | 51.72 mM NaOH | 0.87 |

Table 14 below shows the results of measuring the time required for the reconstitution solution prepared in Examples 45 to 49 to become a transparent solution upon reconstitution, the pH and osmotic pressure of the solution after reconstitution, and the purity of the solution after reconstitution. Here, the purity of the solution after reconstitution was confirmed through UPLC.

**[Table 14]**

| **Example** | **Reconsti tution Time** | **Solution after reconstitution** | | **Peak Area (%)** | | | |
|---|---|---|---|---|---|---|---|
| | | | | **Right after reconstitution** | | **30 min after reconstitution** | |
| | | **pH** | **Osmotic Pressure (mOsm/kg)** | **Active Form** | **Inactive Form** | **Active Form** | **Inactive Form** |
| **Example 45** | Within 1 min | 6.90 | 479 | 95.4 | 4.0 | 94.4 | 5.0 |
| **Example 46** | Within 1 min | 6.91 | 484 | 95.5 | 4.0 | 94.4 | 5.1 |
| **Example 47** | Within 1 min | 6.77 | 372 | 94.5 | 4.5 | 93.1 | 5.8 |
| **Example 48** | Within 1 min | 6.60 | 367 | 94.9 | 4.3 | 93.6 | 5.6 |
| **Example 49** | Within 1 min | 5.85 | 374 | 93.3 | 5.1 | 92.2 | 6.2 |

From the results of Table 14, it was confirmed that the pH of the solution after reconstitution prepared by Examples 45 to 49 was 5.8 to 7.0, which was suitable for use as an injection.

However, the preferred osmotic pressure range of the formulation for use as an injection is 300 to 400 mOsm/kg, and patients may feel pain if it falls below or exceeds this range. Therefore, when these criteria were applied, it was confirmed that Examples 47 and 48, in which the osmotic pressure of the solution after reconstitution was 372 mOsm/kg and 367 mOsm/kg, respectively, were preferable as an injection formulation containing 15 mg/ml API.

In the case of Example 49, the purity was about 93% immediately after reconstitution and about 92% after 30 minutes of reconstitution, so it was confirmed that the API purity itself was too low to be suitable.

### Test Example 9: Preparation of API mixed solution according to stirring device

In the preparation of the API mixed solution, the change in purity of the API in the API mixed solution according to the stirring device was confirmed.

An API mixed solution was prepared using different stirring device, a homogenizer and a magnetic stirrer, according to the API concentration and solvent composition as shown in Table 15 below.

**[Table 15]**

| **Example** | **API Concentration (mg/ml)** | **Solvent Composition** | | | **Stirring Device** |
|---|---|---|---|---|---|
| | | **PB (mM)** | **NaOH (mM)** | **HPβCD(wt%)** | |
| **Example 50** | 15 | 5 | 37.5 | 17.5 | Homogenizer |
| **Example 51** | 15 | 5 | 37.5 | 17.5 | Magnetic stirrer |

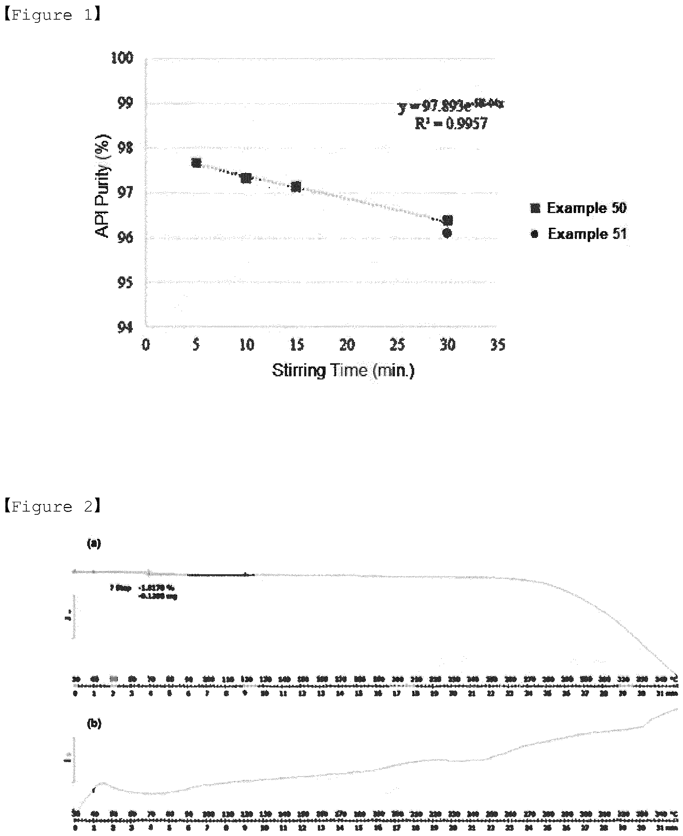

In Example 50 using a homogenizer as a stirring device, it was confirmed that the wettability of the API was not good, but the API and the solvent were mixed well evenly without floating on the solvent. On the other hand, in Example 51 using a magnetic stirrer as a stirring device, the API was not immediately dissolved in the solvent, but floated and took longer to dissolve. Fig. 1 is the results of measuring the API purity in the API mixed solution according to the stirring time. As shown in FIG. 1, in Example 50 using a homogenizer, the purity of the API was 96.4%, and in Example 51 using a magnetic stirrer, the purity of the API was 96.1%, so it was difficult to see that there was an effect on the change in purity according to the stirring device. However, it was confirmed that the purity greatly depended on the dissolution time.

Therefore, it was confirmed that a homogenizer capable of producing a mixed solution quickly and uniformly is suitable as a stirring device.

### Test Example 10: Change in API purity during preparation of freeze-drying formulation

A final API freeze-drying formulation was prepared as described in Table 16 below.

**[Table 16]**

| **Example** | **API conc. (mg/ml)** | **Solvent composition** | | | **stirring Time** | **Acidifying solution** | **Freeze-drying condition** |
|---|---|---|---|---|---|---|---|
| | | **PB** | **NaOH** | **HPβCD** | | | |
| Example 52 | 15 | 5 mM | 37.5 mM | 17.5wt% | 5 min | 50 mM HCl | (Freeze) : - 80°C, 3 hr |
| | | | | | | | (Drying): 12Pa, 48 hr |

In order to confirm the change in purity of the API during the preparation of the final API freeze-drying formulation, the purity of the API powder before dissolving in the solvent composition, and the API purity during the preparation of the API mixture solution and after freeze-drying were measured using UPLC.

**[Table 17]**

| **Condition** | **Purity (%)** |
|---|---|
| API powder | 98.6 |
| API mixed solution | 97.8 |
| After freeze-drying | 97.9 |

In Table 17 above, it was confirmed that the purity mainly decreased in the process of preparing the API mixture solution in the preparation of the final API freeze-drying formulation, and there was little change in the purity in the process of freeze-drying from the API mixture solution.

### Test Example 11: Thermal analysis and XRD pattern analysis of freeze-drying formulation

TGA and DSC of the final API freeze-drying formulation prepared in Example 52 above were measured at a temperature range of 30 to 350°C and a heating rate of 10°C/min.

Fig. 2 is (a) the TGA measurement result of the final API freeze-drying formulation prepared in Example 52 above, and (b) the DSC measurement result of the final API freeze-drying formulation prepared in Example 52 above.

In FIG. 2, it was confirmed that the initial weight loss was estimated as residual moisture and the amount was about 1.8%, and the API was in an amorphous state because no endothermic peak was found.

Therefore, it was confirmed that since the API had a residual moisture of 1.8% (<2%) and was in an amorphous API state, freeze-drying was successful.

The XRD pattern of the final API freeze-drying formulation prepared in Example 52 was measured under conditions of a voltage of 45 kV, a current of 40 mA, and a scan range of 4° to 40°.

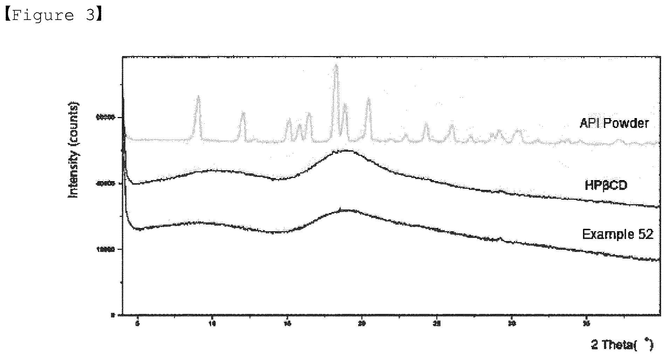

Fig. 3 is the XRD pattern measurement results of the final API freeze-drying formulation prepared in Example 52 above.

In FIG. 3, Example 52, the final API freeze-drying formulation, showed the same pattern as HPβCD, and no API powder peak was found. Therefore, it was confirmed that the API was well dissolved in the solution, well freeze-dried and changed to an amorphous API.

### Test Example 12: Analysis of solid state stability of freeze-drying formulation

In Example 52 above, in order to predict the shelf life of the API freeze-drying formulation in a solid state, Example 52 was stored under the conditions shown in Table 18 below, and then qualitative analysis was performed using UPLC.

**[Table 18]**

| **Temp. (°C)** | **IMP (%Area)** | | | | | |
|---|---|---|---|---|---|---|
| | **1day** | **3days** | **7days** | **14days** | **21days** | **89days** |
| 5 | - | - | - | - | 0.02 | 0.03 |
| 25 | - | - | 0.07 | 0.12 | 0.16 | 0.4 |
| 40 | - | 0.17 | 0.33 | 0.59 | 0.83 | - |
| 50 | 0.16 | 0.42 | 0.89 | - | - | - |
| 60 | 0.22 | 0.65 | - | - | - | - |

In Table 18, the % area value of IMP, which increased the most in the stability test of Example 52, was indicated. It was confirmed that the IMP increased significantly as the storage temperature and time increased.

Therefore, it was confirmed that the API freeze-drying formulation of Example 52 is a stable formulation with a shelf life of about 3 years or less at 5 °C.

## Claims

1. A preparation for injection, comprising:
a first formulation in the powder form including the following compound of Formula 1, a phosphate buffer, a solubilizing agent and a pH adjusting agent; and
a second formulation including a reconstitution solution having a pH of 5 to 12:

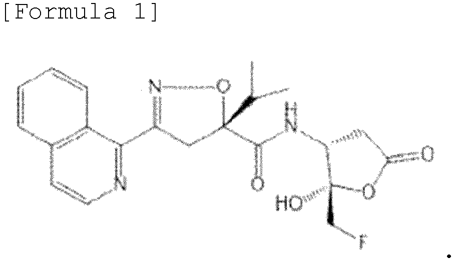

2. The preparation for injection according to claim 1, wherein the phosphate buffer is a mixture of Sodium phosphate dibasic heptahydrate and Sodium phosphate monobasic monohydrate.

3. The preparation for injection according to claim 1, wherein the content of the phosphate buffer is 1 to 15 mM based on the first formulation.

4. The preparation for injection according to claim 1, wherein the solubilizing agent is cyclodextrin.

5. The preparation for injection according to claim 4, wherein the cyclodextrin is any one selected from the group consisting of alpha-cyclodextrin, 2-hydroxypropyl-beta-cyclodextrin and sulfobutyl ether-beta-cyclodextrin.

6. The preparation for injection according to claim 4, wherein the content of the cyclodextrin is 10 to 30 wt% based on the first formulation.

7. The preparation for injection according to claim 1, wherein the pH of the solution after reconstitution by mixing the first formulation and the second formulation is 5.0 to 7.0.

8. The preparation for injection according to claim 1, wherein the osmotic pressure of the solution after reconstitution by mixing the first formulation and the second formulation is 300 mOsm/kg to 400 mOsm/kg.

9. The preparation for injection according to claim 1, wherein the content of the compound of Formula 1 is 1 to 20 mg/ml based on the solution after reconstitution by mixing the first formulation and the second formulation.

10. A method for manufacturing a preparation for injection, comprising the following steps of:
(S1) preparing a solvent having a pH of 10 to 12 including a phosphate buffer, a solubilizing agent and a basic pH adjusting agent;
(S2) preparing a first solution having a pH of 6 to 8 by dissolving the following compound of Formula 1 in the prepared solvent; 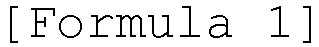 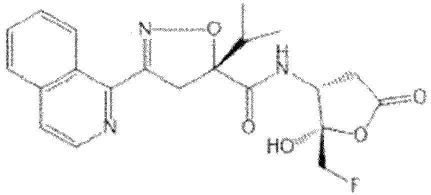
(S3) preparing a second solution having a pH of 1.0 to 3.0 by adding an acidic pH adjusting agent to the first solution;
(S4) preparing a first formulation in the powder form by freeze-drying the second solution; and
(S5) preparing a second formulation including a reconstitution solution having a pH of 5 to 12.

11. The method for manufacturing a preparation for injection according to claim 10, wherein the content of the compound of Formula 1 is 1 to 20 mg/ml based on the solution after reconstitution.

12. The method for manufacturing a preparation for injection according to claim 10, wherein the phosphate buffer is a mixture of Sodium phosphate dibasic heptahydrate and Sodium phosphate monobasic monohydrate.

13. The method for manufacturing a preparation for injection according to claim 10, wherein the content of the phosphate buffer is 1 to 15 mM based on the solvent.

14. The method for manufacturing a preparation for injection according to claim 10, wherein the solubilizing agent is cyclodextrin.

15. The method for manufacturing a preparation for injection according to claim 14, wherein the cyclodextrin is any one selected from the group consisting of alpha-cyclodextrin, 2-hydroxypropyl-beta-cyclodextrin and sulfobutyl ether-beta-cyclodextrin.

16. The method for manufacturing a preparation for injection according to claim 14, wherein the content of the cyclodextrin is 10 to 30 wt% based on the solvent.

17. The preparation for injection according to claim 1, wherein the preparation for injection is for treating or preventing a disease selected from osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer.

18. The preparation for injection according to claim 17, wherein the disease is selected from the group consisting of osteoarthritis, rheumatoid arthritis, degenerative arthritis and destructive bone disorder.

19. The preparation for injection according to claim 1, wherein the preparation for injection is used as a single-dose in a solution state in which the first formulation and the second formulation are mixed.

20. The preparation for injection according to claim 19, wherein the preparation for injection is administered into the joint cavity after mixing the first formulation and the second formulation.

21. The preparation for injection according to claim 19, wherein the preparation for injection is administered into the joint cavity within 30 minutes after mixing the first formulation and the second formulation.

22. The preparation for injection according to claim 19, wherein the preparation for injection is administered once after mixing the first formulation and the second formulation.

23. A preparation for injection, comprising:
a first formulation in the powder form including the following compound of Formula 1, a phosphate buffer, a solubilizing agent and a pH adjusting agent; and
a second formulation including a reconstitution solution having a pH of 5 to 12: 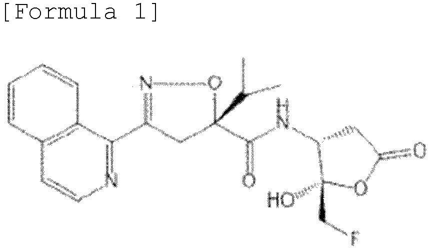
wherein, when the contents of the following compounds of Formula 2 and Formula 3 were measured within 30 minutes after mixing the first formulation and the second formulation, the content of the compound Formula 2 of the total contents of the compounds of Formula 2 and Formula 3 is 94% or more:
